# EUROPEAN PATENT APPLICATION

(11) **EP 4 406 512 A1**
(43) Date of publication of application: **31.07.2024**
(21) Application number: 22872187.4
(22) Date of filing: 26.09.2022
(51) Int. Cl.: A61F 2/24, A61F 2/95, A61M 25/00

(54) **CONVEYING SYSTEM AND PHYSICAL POSITION-LIMITING DEVICE**

(30) Priority: 24.09.2021 CN 202111121584
(71) Applicant: Shanghai Bluesail Boao Medical Technology Co., Ltd., Shanghai 201203 (CN)
(72) Inventor: HERMANUTZ, Lena, 72793 Pfullingen (DE); BAUMGAERTNER, Moritz, 71083 Herrenberg (DE); JIA, Shiqi, Shanghai 201203 (CN); XU, Haiyang, Shanghai 201203 (CN); YIN, Anyuan, Shanghai 201203 (CN)
(74) Representative: Viering, Jentschura & Partner mbB Patent- und Rechtsanwälte
(86) International application number: PCT/CN2022/121244
(87) International publication number: WO 2023/046143

(57) **Abstract**

A conveying system (200) and a physical position-limiting device (100). The device (100) comprises: a first moving component (11); a guide rod (12) moving in the axial direction thereof, at least one stage of position-limiting groove (121) being formed in the outer surface of the guide rod (12), each stage of position-limiting groove (121) comprising first, second, and third sections of groove rail (121a, 121b, 121c) that are sequentially communicated, and the second section of groove rail (121b) being separately arranged at an angle with the first section of groove rail (121a) and with the third section of groove rail (121c); a safety rod (131), which has a first end inserted into the first section of groove rail (121a) and is configured to move relative to the guide rod (12) along the position-limiting groove (121); and a second moving component (14), connected to a second end of the safety rod (131) and configured to drive the safety rod (131) to rotate around the axial direction of the guide rod (12) by means of rotation, such that the first end of the safety rod (131) moves along the second section of groove rail (121b). According to the device (100), an operator can be reminded of a position-limiting critical point by means of touch, accurate information in physical positioning can be effectively fed back, and simple, efficient and accurate physical position-limiting is realized.

## Description

### CROSS-REFERENCE TO RELATED APPLICATION(S)

For all purposes, the present application claims the priority of Chinese Patent Application No.202111121584.4 filed on September 24, 2021, and the disclosure of the above-mentioned Chinese Patent Application is hereby incorporated in its entirety as a part of the present application.

### TECHNICAL FIELD

The present disclosure relates to a conveying system and a physical position-limiting device.

### BACKGROUND

Position limiting generally refers to the limitation of the position of an object. For example, it may be specified that the object is only allowed to be located in a certain area, or it may be specified that the object is not allowed to be located in a certain area. Usually, a position-limiting device can be utilized to accurately determine whether the object reaches a preset state, so as to remind an operator or to achieve an automatic update of state. For example, it is necessary to remind the operator when an object advances and moves to a certain position during conveying. At present, in such scenario of position-limiting reminding, a sensor is commonly utilized for sensing and the operator is reminded of position limiting by means of voice, light or the like, or the operator needs to continuously observe the whole surgical procedure visually to ensure the accuracy of operation and the realization of a preset state. Currently, there is a lack of effective and accurate physical position-limiting device in the industries. Especially, in a conveying system in the field of medical instruments (e.g., the field of conveying of an artificial prosthesis), since an operator of medical instruments often needs to observe the statuses of the patient and of multiple instruments at different positions at the same time, it is difficult to achieve continuous visual attention in the whole surgical procedure as well as the synchronization of visual effect and manual operation for any of these instruments. As a result, there is an urgent need for accurate and effective physical position-limiting device with convenient operation.

### SUMMARY

At least one embodiment of the present disclosure provides a conveying system, a conveying method and a physical position-limiting device.

At least one embodiment of the present disclosure provides a physical position-limiting device including a first movable component, a second movable component, a guiding rod and a safety rod. The guiding rod is configured to be driven by the first movable component to move along an axial direction of the guiding rod, wherein an outer surface of the guiding rod is provided with at least one level of limiting slot, the at least one level of limiting slot extends generally along the axial direction of the guiding rod, each level of limiting slot includes a first groove section, a second groove section and a third groove section which are sequentially communicated, and the second groove section is arranged at an angle relative to the first groove section and the third groove section, respectively. The safety rod includes a first end and a second end which are opposite to each other along a lengthwise direction of the safety rod, wherein the first end of the safety rod is inserted into the limiting slot, and the safety rod is configured to be movable relative to the guiding rod along the limiting slot. The second movable component is connected with the second end of the safety rod, and the second movable component is configured to drive, through rotation, the safety rod to rotate around the axial direction of the guiding rod, so that the first end of the safety rod moves along the second groove section.

For example, in a physical position-limiting device provided by least one embodiment of the present disclosure, the first groove section and the third groove section are configured to extend along the axial direction of the guiding rod, the second groove section is configured to extend along a circumferential direction of the guiding rod, and the second groove section is located between the first groove section and the third groove section in the axial direction of the guiding rod.

For example, in a physical position-limiting device provided by least one embodiment of the present disclosure, the limiting slot further includes a fourth groove section, which is configured for the first end of the safety rod to pass through, wherein a distal end of the fourth groove section is communicated with a proximal end of the third groove section, a proximal end of the fourth groove section is communicated with at least part of the first groove section, and the fourth groove section is not parallel with the second groove section

For example, in a physical position-limiting device provided by least one embodiment of the present disclosure, the first groove section, the second groove section, the third groove section and the fourth groove section surround and delimit a trapezoid or a triangle.

For example, in a physical position-limiting device provided by least one embodiment of the present disclosure, the at least one level of limiting slot includes N levels of limiting slots, where N is an integer greater than or equal to 2, and the first groove section of an i^{th} level of limiting slot is communicated with the third groove section of an (i-1)^{th} level of limiting slot, wherein a value i of each level is sequentially marked as 1, 2 ..., N from a proximal end to a distal end.

At least one embodiment of the present disclosure provides a conveying system for an artificial prosthesis, including the physical position-limiting device described in any of the above. The conveying system further includes a first tube assembly and a second tube assembly. The first tube assembly is configured for an artificial prosthesis to be placed therein; and the second tube assembly includes a sheath tube, wherein the sheath tube is sleeved on an outer side of at least part of the first tube assembly, and axial directions of the sheath tube and the first tube assembly are parallel to or coaxial with the axial direction of the guiding rod respectively. The sheath tube is fixedly connected with the first movable component, so that the first movable component drives the sheath tube to move axially relative to the first tube assembly.

For example, in a conveying system provided by least one embodiment of the present disclosure, the safety rod is immovable relative to the first tube assembly.

For example, a conveying system provided by least one embodiment of the present disclosure further includes a screw, wherein the first movable component includes a first knob which is sleeved on the screw, and axial directions of the first knob and the screw are both parallel to or coaxial with the axial direction of the guiding rod. An interior of the first knob is provided with a screw thread matched with the screw to obtain a threaded fit so that the first knob carries out a relative movement along an axial direction of the first tube assembly through screw drive in a case that the first knob rotates in a circumferential direction.

For example, a conveying system provided by least one embodiment of the present disclosure further includes a first half shell, a second half shell, a third half shell and a fourth half shell. A distal end of the screw is fixedly connected with the first half shell and the second half shell, respectively, and a proximal end of the screw is fixedly connected with the third half shell and the fourth half shell, respectively. The first half shell and the second half shell are located at two sides of a central axis of the conveying system, respectively, and are fixedly connected to form a first housing; the third half shell and the fourth half shell are located at two sides of the central axis of the conveying system, respectively, and are fixedly connected to form a second housing.

For example, in a conveying system provided by least one embodiment of the present disclosure, the second tube assembly further includes a stabilizer tube, and a distal end of the stabilizer tube is sleeved on an outer side of at least part of the sheath tube.

For example, in a conveying system provided by least one embodiment of the present disclosure, the sheath tube is a reducer tube including a first sheath tube portion and a second sheath tube portion which are sequentially arranged from a distal end to a proximal end. A diameter of the first sheath tube portion is larger than that of the second sheath tube portion, a distal end of the stabilizer tube is sleeved on an outer side of the second sheath tube portion, and a diameter of the stabilizer tube is smaller than that of at least part of the first sheath tube portion.

For example, a conveying system provided by least one embodiment of the present disclosure further includes a stabilizer tube holder and a sheath tube holder. The stabilizer tube is configured to extend from a distal end to the stabilizer tube holder, and a proximal end of the stabilizer tube is fixedly connected with the stabilizer tube holder; the stabilizer tube holder is arranged inside the first housing, and the stabilizer tube holder is fixedly connected with the first half shell and the second half shell, respectively. The sheath tube extends from a distal end to the sheath tube holder, a proximal end of the second sheath tube portion is fixedly connected with the sheath tube holder, and the sheath tube holder is fixedly connected with the first knob. The guiding rod passes through the screw and is fixedly connected with the sheath tube holder, so that the sheath tube holder can be driven to move by means of a rotation of the first knob, and that the sheath tube and the guiding rod can be driven by means of the rotation of the first knob to move relative to each other along an axial direction of the first tube assembly.

For example, in a conveying system provided by least one embodiment of the present disclosure, the first tube assembly includes an inner tube and an artificial prosthesis connector, the artificial prosthesis connector is arranged on an outer surface of a distal end of the inner tube and is fixedly connected with the inner tube; the artificial prosthesis connector is provided with a clamping slot matched with the artificial prosthesis for the artificial prosthesis to be embedded in the clamping slot, so that the artificial prosthesis is detachably connected with the artificial prosthesis.

For example, a conveying system provided by least one embodiment of the present disclosure further includes an inner tube holder. The inner tube is configured to extend proximally from a distal end to the inner tube holder and is fixedly connected with the inner tube holder, wherein the inner tube holder is arranged inside the second housing, and the inner tube holder is fixedly connected with the third half shell and the fourth half shell, respectively.

For example, in a conveying system provided by least one embodiment of the present disclosure, the inner tube includes a plurality of third tube bodies which are sequentially connected along an axial direction of the first tube assembly and have different hardness, and the sheath tube includes a plurality of fourth tube bodies which are sequentially connected along the axial direction of the first tube assembly and have different hardness.

For example, in a conveying system provided by least one embodiment of the present disclosure, the first tube assembly further includes an inner tube evacuator and an end base, wherein the inner tube evacuator is connected with a proximal end of the inner tube, and the end base is connected with the distal end of the inner tube.

For example, a conveying system provided by least one embodiment of the present disclosure further includes a safety connector, wherein the second movable component is a second knob, and an axial direction of the second knob is parallel to or coaxial with the axial direction of the guiding rod; the safety connector is fixedly connected with the second end of the safety rod and the second knob, respectively, so that the safety rod can be driven to rotate around the axial direction of the guiding rod by means of a rotation of the second knob.

For example, in a conveying system provided by least one embodiment of the present disclosure, the second housing is a cylinder, and the second knob is sleeved on an outer surface of the cylinder, so that the second knob cannot move along the axial direction of the guiding rod and can rotate around the axial direction of the guiding rod.

For example, in a conveying system provided by least one embodiment of the present disclosure, the artificial prosthesis includes an artificial heart valve, a stent graft or an artificial blood vessel.

For example, in a conveying system provided by least one embodiment of the present disclosure, at least part of the artificial prosthesis is placed in a cavity between the sheath tube of the second tube assembly and the first tube assembly in a case that the first end of the safety rod is inserted into the first groove section, and the cavity is gradually opened or closed in a case that the sheath tube of the second tube assembly moves axially relative to the first tube assembly, thereby releasing the artificial prosthesis or recovering the artificial prosthesis.

For example, in a conveying system provided by least one embodiment of the present disclosure, a cavity between the sheath tube of the second tube assembly and the first tube assembly in which the artificial prosthesis is placed in a case that the first end of the safety rod is located at a proximal end inside the first groove section is a first cavity. A cavity between the sheath tube of the second tube assembly and the first tube assembly in which a part of the artificial prosthesis is placed in a case that the first end of the safety rod is limited at a distal end inside the first groove section is a second cavity. A space of the second cavity is smaller than that of the first cavity.

At least one embodiment of the present disclosure provides a conveying method for an artificial prosthesis, including: controlling a first movable component to move, so as to drive a guiding rod to move along an axial direction of the guiding rod, and to drive a sheath tube of a second tube assembly to move axially relative to a first tube assembly, wherein the sheath tube of the second tube assembly is fixedly connected with the first movable component, the first tube assembly is configured for an artificial prosthesis to be placed therein, the sheath tube of the second tube assembly is sleeved on an outer side of at least part of the first tube assembly, axial directions of the sheath tube of the second tube assembly and the first tube assembly are respectively parallel to or coaxial with the axial direction of the guiding rod, an outer surface of the guiding rod is provided with at least one level of limiting slot which extends generally along the axial direction of the guiding rod, and each level of limiting slot includes a first groove section, a second groove section and a third groove section which are communicated in sequence, and the second groove section is arranged at an angle relative to the first groove section and the third groove section respectively; inserting a first end of a safety rod into the first groove section, and moving the guiding rod proximally along the axial direction so that the first end of the safety rod can be limited at one end (for example, a distal end inside the first groove section) inside the first groove section close to the second groove section; controlling a rotation of the second movable component to drive the safety rod to rotate around the axial direction in response to the artificial prosthesis satisfying a target requirement, so that the first end of the safety rod is rotated from the first groove section to at least part of the third groove section through the second groove section, and that the guiding rod is driven by the first movable component to move proximally along the axial direction in a state where the third groove section allows the first end of the safety rod to pass through, and that the sheath tube of the second tube assembly is driven by the first movable component to move axially relative to the first tube assembly, wherein the second movable component is fixedly connected with the safety rod.

For example, a conveying method provided by at least one embodiment of the present disclosure further includes: controlling the first movable component to move reversely in response to the artificial prosthesis failing to satisfy the target requirement, so as to drive the sheath tube of the second tube assembly to move axially relative to the first tube assembly and to drive the guiding rod to move distally along the axial direction.

For example, a conveying method provided by at least one embodiment of the present disclosure further includes: controlling the first movable component to move reversely in response to the first end of the safety rod being close to the distal end inside the third groove section, to drive the sheath tube of the second tube assembly to move axially relative to the axial direction of the first tube assembly and to drive the guiding rod to move distally along the axial direction, so that the first end of the safety rod returns to the first groove section from the third groove section through a fourth groove section of the limiting slot, wherein a first end of the fourth groove section is communicated with a proximal end of the third groove section, a second end of the fourth groove section is communicated with at least part of the first groove section, and the fourth groove section is not parallel with the second groove section, so as to allow the first end of the safety rod to move proximally along the fourth groove section.

For example, in a conveying method provided by at least one embodiment of the present disclosure, the conveying method further includes: in a case that the first end of the safety rod is inserted into the first groove section, placing at least part of the artificial prosthesis in a cavity between the sheath tube of the second tube assembly and the first tube assembly, and driving the sheath tube of the second tube assembly to move axially relative to the first tube assembly, so that the cavity is gradually opened or closed to release the artificial prosthesis or recover the artificial prosthesis.

Compared with the prior art, the beneficial effects of at least one embodiment of the present disclosure at least include: the device or method of the embodiments of the present disclosure can achieve physical limiting of position, so that the operator can be reminded of position-limiting critical point through tactile sensation, and accurate information in physical positioning can be effectively fed back, thereby achieving a simple, highly efficient and accurate physical limiting of position.

### BRIEF DESCRIPTION OF DRAWINGS

In order to explain the technical solution in the embodiments of the present disclosure or in the prior art more clearly, the drawings necessary for the description of the embodiments or the prior art will be briefly introduced below. Obviously, the drawings in the following description are only some embodiments of the present disclosure, and other drawings can be obtained according to these drawings without inventive steps for those ordinary skilled in the art.
Fig. 1 is a schematic structural diagram of a physical position-limiting device provided by some embodiments of the present disclosure;
Figs. 2 to 3 are schematic diagrams of an outer surface of a guiding rod provided by some embodiments of the present disclosure from different perspectives;
Fig. 4a is a simplified schematic diagram of Fig. 1 with a safety device and a second movable component being remained, from a perspective of viewing from a proximal end towards a distal end in an axial direction, as provided by some embodiments of the present disclosure;
Fig. 4b is a cross-sectional view of Fig. 4a provided by some embodiments of the present disclosure;
Figs. 5a to 5f are schematic diagrams of an operation method of a physical position-limiting device provided by some embodiments of the present disclosure;
Fig. 6 is a schematic diagram illustrating a cascade connection of two levels of limiting slots provided by some embodiments of the present disclosure;
Fig. 7 is a schematic diagram of a conveying system for an artificial prosthesis provided by some embodiments of the present disclosure;
Fig. 8 is a partial schematic diagram of a first tube assembly and a second tube assembly provided by some embodiments of the present disclosure;
Fig. 9 is an axial cross-sectional view of a conveying system for an artificial prosthesis provided by some embodiments of the present disclosure;
Figs. 10a to 10c are schematic diagrams of an operation method of a conveying system for an artificial prosthesis provided by some embodiments of the present disclosure.

### DETAILED DESCRIPTION

In the following, the technical solution(s) in the embodiments of the present disclosure will be clearly and completely described with reference to the accompanying drawings of the embodiments of the present disclosure. Obviously, the described embodiments are only a part of but not all of the embodiments of the present disclosure. Based on the embodiments in the present disclosure, all other embodiments obtained by those ordinary skilled in the art without inventive steps belong to the scope of protection of the present disclosure.

Unless otherwise defined, all terms (including technical and scientific terms) used in the embodiments of the present disclosure have the same meaning as commonly understood by those ordinary skilled in the art to which the present disclosure belongs. It should also be understood that terms such as those defined in general dictionaries should be interpreted as having meanings consistent with their meanings in the context of the related art, and should not be interpreted in an idealized or extremely formal sense, unless explicitly defined by the embodiment of the present disclosure.

The words "first", "second" and similarities used in the embodiments of the present disclosure do not indicate any order, quantity or importance, but are only used to distinguish different components. Similar words such as "a", "an" or "the" do not mean a quantity limit, but mean that there is at least one. Also, similar words such as "including" or "containing" mean that the elements or objects appearing before the word cover the elements or objects listed after the word and their equivalents, without excluding other elements or objects. Similar words such as "connected" or "connecting" are not limited to physical or mechanical connection, but can include electrical connection, whether direct or indirect. A flowchart is used in the embodiment of the present disclosure to explain the steps of the method according to the embodiment of the present disclosure. It should be understood that the preceding or subsequent steps are not necessarily performed accurately in sequence. Instead, the various steps can be processed in reverse order or simultaneously. At the same time, other operations can also be added to these processes, or a step or steps can be removed from these processes.

The inventor(s) found that with the aging of the population, the incidence of valvular heart disease is increased significantly. Traditional treatment methods include conservative drug therapy and surgical valve replacement. Drug therapy has little help in prognosis improvement, while surgical valve replacement can obviously improve the prognosis of patients. However, for many elderly patients with a medical history of thoracotomy and severe cardiopulmonary dysfunction, the surgery involves higher risk, and even the opportunity for a surgery may be missed. Under such conditions, transcatheter aortic valve replacement is a monumental development in the diagnosis and treatment of valvular heart disease in recent years.

During the implantation process of an artificial heart valve in such surgery, an operator needs to accurately know the release position and release form of the valve prosthesis. In the process of determining the position of the valve prosthesis, interference factors such as unclear angiography and unstable operation make it even more difficult to locate the valve prosthesis. Moreover, in the case where a conventional artificial heart valve implantation device is used, once the operator completes the positioning and begins to release the valve prosthesis, the whole release process is irreversible, and the operator has almost no chance to adjust the position and form of the prosthesis, which results in an extremely low fault tolerance rate of the operation process. Therefore, it is extremely difficult for the operator to know the length of the valve released from the conveying system in real time.

There are mainly two ways for the operator to know the length of the valve released from the conveying system. The first way is to determine the release condition of the prosthesis by comparing the relative positions of the development markers and the valve prosthesis with the aid of ultrasonic images and digital subtraction angiography. In this method, the operator cannot accurately control the release length of the valve due to the delay of development. The second way is to obtain the information of release length of the valve by reading the release stroke mark on the handle or tubing. In this method, since there is a certain included angle between the operator's observation position and the stroke mark during the surgical procedure, a parallax is caused in the reading process, and the operator cannot know the accurate information of the release length of the valve either.

In view of this, the present disclosure provides a simple, highly efficient and accurate physical position-limiting device, as well as a system and a method of using the physical position-limiting device.

At least one embodiment of the present disclosure provides a physical position-limiting device, including: a first movable component; a guiding rod configured to be driven by the first movable component to move along an axial direction of the guiding rod, wherein an outer surface of the guiding rod is provided with at least one level of limiting slot, the at least one level of limiting slot extends generally along the axial direction of the guiding rod, each level of limiting slot includes a first groove section, a second groove section and a third groove section which are sequentially communicated, and the second groove section is arranged at an angle relative to the first groove section and the third groove section respectively; a safety rod including a first end and a second end which are opposite to each other along a lengthwise direction of the safety rod, wherein the first end of the safety rod is inserted into the limiting slot, and the safety rod is configured to be movable relative to the guiding rod along an extension direction of the limiting slot; and a second movable component connected with the second end of the safety rod, wherein the second movable component is configured to drive, through rotation, the safety rod to rotate around the axial direction of the guiding rod, so that the first end of the safety rod moves along the second groove section.

At least one embodiment of the present disclosure also provides a method corresponding to the physical position-limiting device described above.

The physical position-limiting device or the method in the above embodiments of the present disclosure can achieve physical limiting of position, so that the operator can be reminded of position-limiting critical point through tactile sensation, and accurate information in physical positioning can be effectively fed back, thereby achieving a simple, highly efficient and accurate physical limiting of position.

Hereinafter, embodiments of the present disclosure and examples thereof will be described in details with reference to the accompanying drawings.

It should be noted that, for the convenience of description here, in some embodiments of the present disclosure, a first side of an axial direction is labeled as a left side in the drawings, and a second side of the axial direction is labeled as a right side in the drawings. For example, a direction perpendicular to the axial direction in some embodiments of the present disclosure can be labeled as an up-and-down direction in the drawings, but the up-and-down direction involved in the embodiments of the present disclosure merely represents an orientation in the drawings without affecting the orientation in practical application, which is not limited in the embodiments of the present disclosure.

For example, regarding the definition of the axial direction, for convenience of description, in at least one embodiment of the present disclosure, the side close to the operator may be considered as a proximal end or a proximal side, and the side away from the operator may be considered as a distal end or a distal side. For example, the left side in the drawings may be considered as the distal end/side, and the right side in the drawings may be considered as the proximal end/side. It should be noted that the distal end, the proximal end and the like of the present disclosure are all relative positions, for example, representing two opposite sides of some components themselves, or representing two opposite sides in a certain direction; that is, in the present disclosure, the proximal end represents one side, and the distal end represents the other side opposite to the proximal end. The meanings and functions of elements or objects in the embodiments of the present disclosure are neither restricted to their names nor interpreted in an idealized or extremely formal sense, which will not constitute any limitation to the embodiments of the present disclosure.

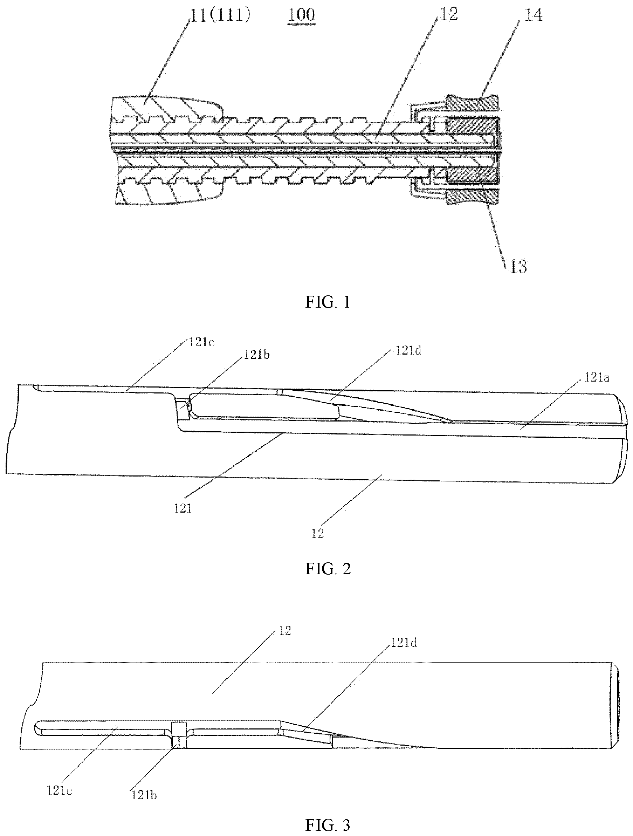

Fig. 1 is a schematic structural diagram of a physical position-limiting device 100 provided by some embodiments of the present disclosure.

As shown in Fig. 1, the physical position-limiting device 100 includes a first movable component 11, a guiding rod 12, a safety device 13 and a second movable component 14. The first movable component 11 is configured to drive the guiding rod 12 so that the guiding rod 12 moves along an axial direction of the guiding rod 12. For example, the axial direction of the guiding rod 12 can be regarded as an axial direction of the whole physical position-limiting device 100.

In some examples, the first movable component 11 may be a rotatable component that rotates around the axial direction of the guiding rod 12. For example, the first movable component 11 converts a rotation into a linear motion through an intermediate component so as to drive the guiding rod 12 to move along the axial direction of the guiding rod 12. For example, as shown in Fig. 1, the first movable component 11 is a first knob 111 (for example, a manual knob). The axial direction of the first knob 111 is parallel to or coaxial with the axial direction of the guiding rod 12, and the first knob 111 is configured to drive the guiding rod 12 to move along the axial direction of the guiding rod 12 through a rotation of the first knob 111 itself (see the following description for specific examples).

The structure of the first movable component 11 is not limited to this, and any component or assembly that can cause the guiding rod 12 to move along the axial direction of the guiding rod 12 belongs to the scope of protection of the embodiments of the present disclosure. For example, the first movable component 11 may be a component that moves linearly along the axial direction.

Fig. 2 and Fig. 3 are schematic perspective views of an outer surface of the guiding rod 12 provided by some embodiments of the present disclosure from different perspectives. Fig. 3 is a schematic diagram illustrating a state of the guiding rod 12 of Fig. 2 after rotating around the axial direction for a certain angle.

As shown in Fig. 2 and Fig. 3, the outer surface of the guiding rod 12 is provided with at least one level of limiting slot 121, and the at least one level of limiting slot 121 extends generally along the axial direction of the guiding rod 12. Each level of limiting slot 121 includes a first groove section 121a, a second groove section 121b and a third groove section 121c which are communicated in sequence. The second groove section 121b is arranged at an angle with respect to the first groove section 121a and the third groove section 121c, respectively.

It should be noted that the overall extension of the limiting slot 121 along the axial direction of the guiding rod 12 does not mean that the first groove section 121a, the second groove section 121b and the third groove section 121c all extend along the axial direction, but that the extension direction of the integrated structure formed by the sequential communication of the first groove section 121a, the second groove section 121b and the third groove section 121c is along the axial direction of the guiding rod 12, in which any one of the first groove section 121a, the second groove section 121b and the third groove section 121c may not extend along the axial direction of the guiding rod 12 but extend substantially along the axial direction of the guiding rod 12.

As shown in Figs. 2 and 3, the first groove section 121a and the third groove section 121c are configured to extend along a direction parallel to the axial direction of the guiding rod 12. The second groove section 121b is configured to extend along a circumferential direction of the guiding rod 121. In the axial direction of the guiding rod 121, the second groove section 121b is located between the first groove section 121a and the third groove section 121c.

For example, the first groove section 121a, the second groove section 121b and the third groove section 121c are all slots with a certain depth formed in an external circumferential surface of the guiding rod 12, and parameters such as a cross-sectional shape and a depth of each groove section are not limited here.

In some examples, the shape of any section of the limiting slot 121 in the extension direction may not be a straight line in the strict sense, for example, it is basically a straight line. For another example, the extension direction of the second groove section 121b may not be the circumferential direction of the guiding rod 121 in the strict sense, for example, it may be basically consistent with the circumferential direction of the guiding rod 121.

In at least one embodiment of the present disclosure, the second groove section 121b is arranged at an angle with respect to the first groove section 121a and the third groove section 121c respectively, which means that on the external circumferential surface of the guiding rod 12, the second groove section 121b is a section of groove that is neither parallel to the first groove section 121a nor parallel to the third groove section 121c.

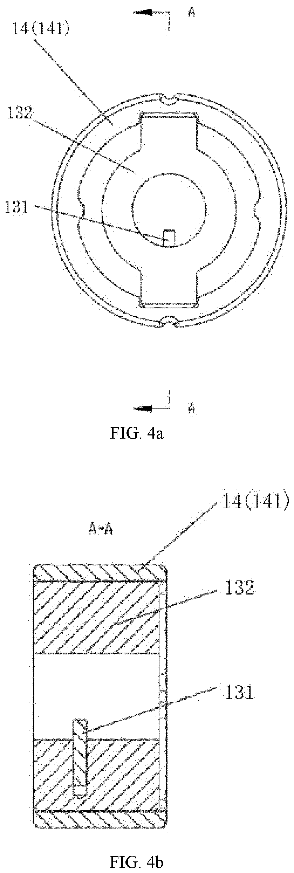

Fig. 4a is a simplified schematic diagram of Fig. 1 with a safety device 13 and a second movable component 14 being remained, from a perspective of viewing from a proximal end towards a distal end in an axial direction, as provided by some embodiments of the present disclosure. Fig. 4b is a cross-sectional view taken along section line A-A of Fig. 4a.

As shown in Figs. 4a and 4b, the safety device 13 includes a safety rod 131, the safety rod 131 includes a first end (i.e., the end of the safety rod 131 close to the guiding rod 12) and a second end (i.e., the end of the safety rod 131 away from the guiding rod 12) which are arranged opposite to each other along a lengthwise direction of the safety rod. The first end of the safety rod 131 is inserted into the first groove section 121a of the limiting slot 121. The safety rod 131 is configured to be movable relative to the guiding rod 12 along an extension direction of the limiting slot 121.

In some examples, the safety rod 131 is immovable and is movable relative to the guiding rod 12, that is, the relative sliding of the safety rod 131 in different sections of the groove of the limiting slot 121 of the guiding rod 12 is resulted by the guiding rod 12 moving proximally (or moving distally) while the safety rod 131 being immovable, which allows the safety rod 131 to move distally (or proximally) relative to the guiding rod 12 correspondingly. Therefore, according to the present disclosure, the physical limiting of position of the guiding rod is achieved by configuring the immovability of the safety rod 131 to be immovable but have a relative movement with respect to the guiding rod, which enables simple structure, convenient operation as well as good stability and accuracy.

It should be noted that the safety rod 131 may be immovable with respect to the current operator, or may be immovable with respect to the current environment in which the whole conveying system is located.

Therefore, the first groove section 121a and the third groove section 121c are configured to allow the first end of the safety rod 131 to pass through, so as to allow the guiding rod 12 to smoothly move along the axial direction of the guiding rod, for example, the guiding rod 12 moves proximally along the axial direction of the guiding rod (i.e., moving to the right side along the axial direction); at this time, the safety rod 131 has a relative movement of passing through the first groove section 121a and the third groove section 121c, from a proximal end to a distal end of the axial direction.

For example, based on the guiding rod 12 moving proximally along the axial direction, the first end of the safety rod 131 can move relative to the guiding rod 12 to reach the distal end from the proximal end inside the first groove section 121a, and can be limited at an end inside the first groove section 121a close to the second groove section 121b (that is, the distal end inside the first groove section 121a).

In some examples, the second end of the safety rod 131 is fixedly connected with the second movable component 14. The safety rod 131 is configured to be driven to rotate around the axial direction of the physical position-limiting device 100 by means of a rotation of the second movable component 14, so that the first end of the safety rod 131 is rotated from the first groove section 121a to the third groove section 121c via the second groove section 121b of the limiting slot 121.

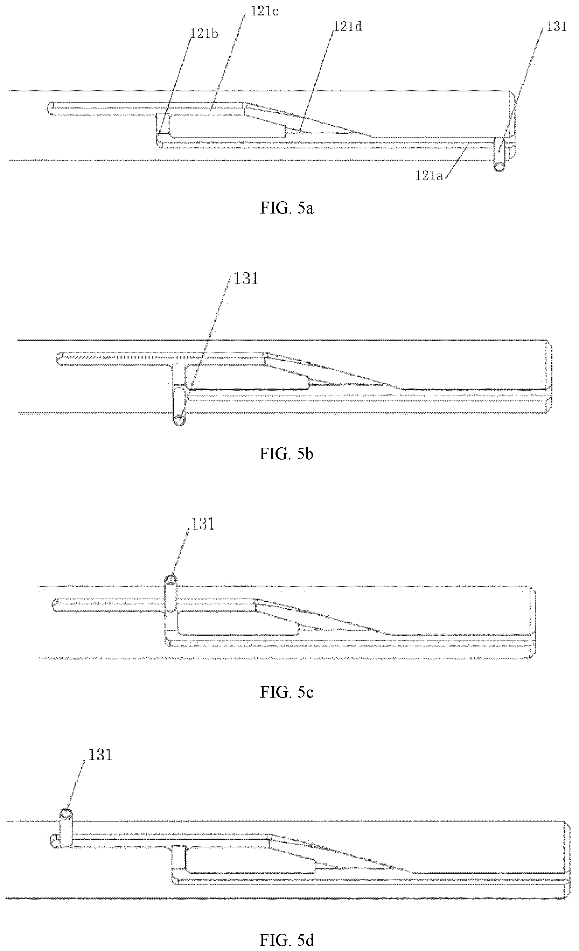

As shown in Fig. 2, the limiting slot 121 further includes a fourth groove section 121d. The third groove section 121c, the fourth groove section 121d and the first groove section 121a are sequentially communicated, and the fourth groove section 121d is arranged at an angle with respect to the third groove section 121c and the first groove section 121a, respectively. The distal end of the fourth groove section 121d is communicated with the proximal end of the third groove section 121c, and the fourth groove section 121d extends to a side away from the second groove section 121b, so that the proximal end of the fourth groove section 121d is communicated with at least part of the first groove section 121a, and the fourth groove section 121d is not parallel to the second groove section 121b, so as to allow the first end of the safety rod 131 to move proximally along the fourth groove section 121b. Therefore, the fourth groove section 121d has a chute shape, e.g., a chute inclined from top left to bottom right as shown in Fig. 5a, which is convenient for recycling.

For example, the distance between the proximal end of the chute-shaped fourth groove section 121d and the straight line where the second groove section 121b is located is smaller than the distance between the proximal end of the fourth groove section 121d and the straight line where the second groove section 121b is located.

In some examples, the fourth groove section 121d is configured to allow the first end of the safety rod 131 to pass through, so as to allow the guiding rod 12 to smoothly move along the axial direction of the guiding rod, for example, the guiding rod 12 moves distally along the axial direction of the guiding rod (that is, moving to the left side along the axial direction); at this time, the safety rod 131 has a relative movement of passing through the fourth groove section 121d from a distal end to a proximal end of the axial direction inside the fourth groove section 121d, thereby realizing the recovering function of the physical position-limiting device 100.

As shown in Fig. 2, the first groove section 121a, the second groove section 121b, the third groove section 121c and the fourth groove section 121 of the limiting slot 121 surround and delimit a trapezoid, so that the limiting slot 121 is in the shape of a Chinese character " 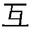 ", as a whole. This is merely exemplary and is not a limitation of the present disclosure.

As shown in Figs. 4a and 4b, the safety device 13 also includes a safety connector 132, the second end of the safety rod 131 is fixedly connected with the safety connector 132, and the safety connector 132 is also fixedly connected with the second movable component 14, so that the safety rod 131 can be driven to rotate around the axial direction of the physical position-limiting device 100 through a movement of the second movable component 14.

As shown in Figs. 4a and 4b, the second movable component 14 includes a second knob 141, for example, the second knob 141 is a manual knob, and the axial direction of the second knob 141 is parallel to or coaxial with the axial direction of the guiding rod 12.

In some examples, the second knob 141 and the safety device 13 are fixedly connected by means of a form fit or an interference fit. This is merely exemplary and is not a limitation of the present disclosure.

Hereinafter, an operation method of the physical position-limiting device of the embodiment of the present disclosure will be described with reference to Figs. 5a-5f which illustrate state diagrams.

As shown in Fig. 5a, the first end of the safety rod 131 is inserted into the right end of the first groove section 121a on the outer surface of the guiding rod 12. The first movable component 11 is controlled to move, so as to drive the guiding rod 12 to move proximally along the axial direction. At this time, the safety rod 131 has a relative movement, gradually, from the proximal end of the first groove section 121a to the distal end of the first groove section 121a, as shown in Fig. 5b. In this state, the safety rod 131 reaches the limiting position of the first groove section 121a. Due to the blocking effect of the inner wall at the distal end of the first groove section 121a, the safety rod 131 can no longer move forward relative to the guiding rod 12 along with the movement of the guiding rod 12. That is, the first movable component 11 and the guiding rod 12 cannot continue to move in the axial direction because they are limited by the safety rod 131 inside the first groove section 121a, thus achieving the function of physical limiting of position. Therefore, the operator can be reminded of position-limiting critical point through tactile sensation of the first movable component, and accurate information in physical positioning can be effectively fed back, thereby achieving a simple, highly efficient and accurate physical limiting of position.

For example, after the position limiting is achieved, by rotating the second movable component 14 around the axial direction of the physical position-limiting device 100, the first end of the safety rod 131 is rotated from the distal end of the first groove section 121a (i.e., the end of the second groove section 121b close to the first groove section 121a) to the inside of the third groove section 121c (e.g., rotated to the proximal end or the middle position of the third groove section 121c) via the second groove section 121b. This means that the relative movement in the axial direction between the safety rod 131 and the guiding rod 12 is unlocked, as shown in Fig. 5c. In this state, under the drive of the first movable component 11, the guiding rod 12 can continue to move proximally along the axial direction, and then the first end of the safety rod 131 slides, relative to the guiding rod 12, inside the third groove section 131c, from the proximal end of the third groove section 121c to the distal end of the third groove section 121c along the axial direction, as shown in Fig. 5d.

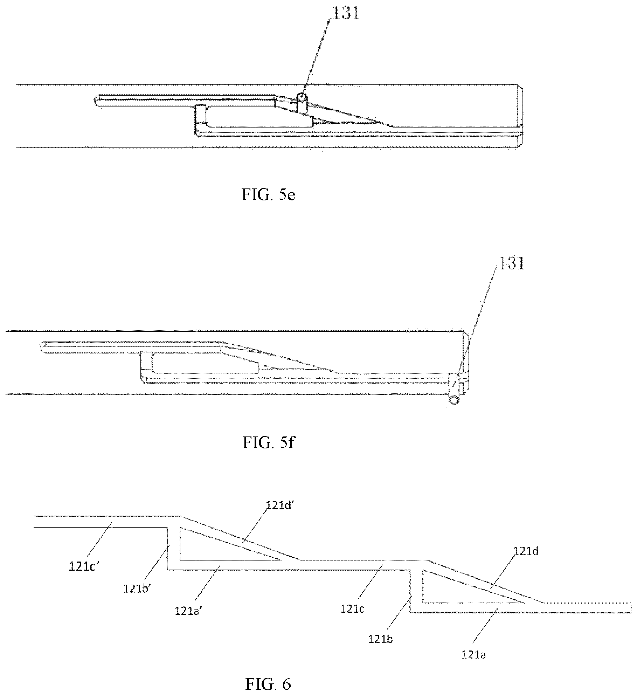

For example, in a returning level (which can also be referred to as a recovering level) after the safety rod 131 reaching the distal end of the third groove section 121c, under the drive of the first movable component 11 which moves reversely, the guiding rod 12 also moves reversely, i.e., the guiding rod 12 moves distally along the axial direction, so that the safety rod 131 slides proximally relative to the guiding rod 12 inside the third groove section 121c along the axial direction. The safety rod 131 reaches the proximal end of the third groove section 121c firstly, and then transits to the distal end inside the chute-shaped fourth groove section 121d, as shown in Fig. 5e. The safety rod 131 continues to slide relative to the guiding rod 12 inside the fourth groove section 121d until it moves to the proximal end inside the fourth groove section 121d, then the safety rod 131 transits to the distal end inside the first groove section 121a and slides relative to the guiding rod 12 towards the distal end of the first groove section 121a until it finally reaches the proximal end of the first groove section 121d, thus completing the returning level, as shown in Fig. 5f.

Therefore, in at least one embodiment of the present disclosure, the limiting slot 121 includes a chute-shaped fourth groove section 121d, so that the second movable component 14 can be passively driven to rotate to the initial position (i.e., reset) merely by operating the first movable component 11 (e.g., the first knob 111) without additionally operating the second movable component 14 (i.e., the second knob 141), which makes the whole operating process more convenient and efficient.

It should be noted that the physical position-limiting device of the embodiments described above with reference to Figs. 1 to 5f only involves one level of limiting slot 121, but the present disclosure is not limited to this, and the physical position-limiting device of the present disclosure may also include two or more levels of limiting slots 121.

For example, the physical position-limiting device of the embodiment of the present disclosure may include N level of limiting slots 121, where N is an integer greater than or equal to 2, and the first groove section 121a of an i^{th} level of limiting slot 121 is communicated with the third groove section 121c of an (i-1)^{th} level of limiting slot 121, wherein the value of each level i may be sequentially marked as 1, 2 ... N, respectively, from the proximal end to the distal end of the axial direction.

Fig. 6 is a schematic diagram illustrating a cascade connection of two levels of limiting slots 121 provided by some embodiments of the present disclosure.

For example, as shown in Fig. 6, the first level of limiting slot in the two levels of limiting slots 121 includes a first groove section 121a, a second groove section 121b, a third groove section 121c and a fourth partial groove section 121d. The second level of limiting slot includes a first groove section 121a', a second groove section 121b', a third groove section 121c' and a fourth groove section 121d'. The left end of the third groove section 121c of the first level is communicated with or integrally formed with the right end of the first groove section 121a' of the second level, so as to form the cascade connection of the two levels of limiting slots 121. In the embodiment of the present disclosure, for the cascade connection mode as well as specific structure and construction of the limiting slots 121 with more than three levels, reference can be made to the example of Fig. 6, which will not be repeated here.

In some examples, the overall shape and structure of the limiting slot of each level can be adjusted as required. For example, the limiting slot is not limited to the trapezoid shown in Fig. 2. Alternatively, as shown in Fig. 6, the first groove section 121a, the second groove section 121b, the third groove section 121c and the fourth groove section 121 of the limiting slot 121 can also surround and delimit a triangle. This is merely exemplary and is not a limitation of the present disclosure.

For example, in the example of Fig. 6, each level of limiting slot in the two levels of limiting slots 121 has the same structure. Of course, the present disclosure is not limited to this, for example, the structures of the two levels of limiting slots 121 may also be different.

In some examples, some levels of limiting slots 121 among multiple levels of limiting slots 121 may be in trapezoidal shape, and the other levels of limiting slots 121 may be in triangular shape. In some other examples, each level of limiting slot 121 among the multiple levels of limiting slots 121 is in trapezoidal shape. In yet some examples, each level of limiting slot 121 among the multiple levels of limiting slots 121 is in triangular shape.

Therefore, at least one embodiment of the present disclosure can achieve position limiting at multiple positions or multiple levels through the cascade connection of multiple levels of limiting slots, merely by using one safety device 13 (e.g., the safety rod 131) and one second movable component 14 (e.g., the second knob 141), which is very simple and highly efficient, wider in application range and higher in accuracy of position limiting.

At least one embodiment of the present disclosure also provides a conveying system for an artificial prosthesis, which includes the physical position-limiting device of any of the above embodiments.

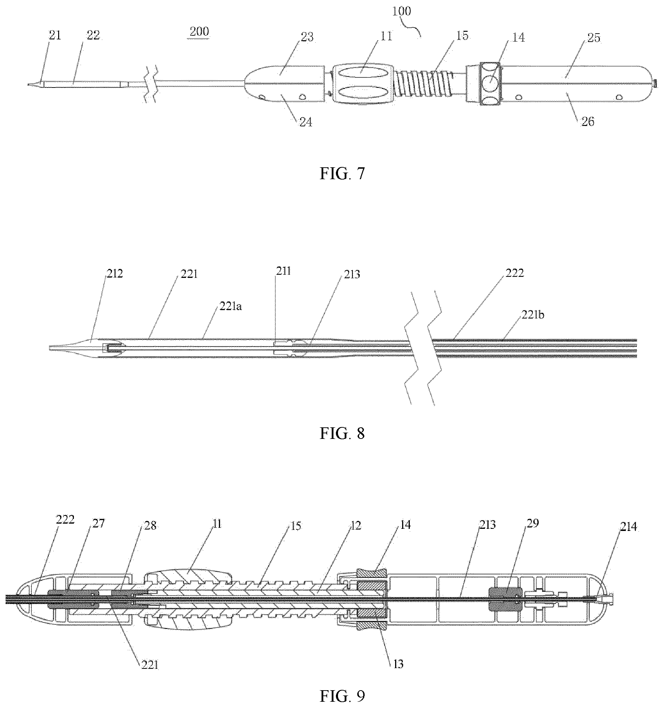

Fig. 7 is a schematic diagram illustrating an appearance of a conveying system for an artificial prosthesis provided by some embodiments of the present disclosure. Fig. 8 is a structural diagram of a first tube assembly and a second tube assembly provided by some embodiments of the present disclosure, and Fig. 9 is a schematic diagram illustrating an interior of a conveying system for an artificial prosthesis provided by some embodiments of the present disclosure.

As shown in Fig. 7, the conveying system 200 for an artificial prosthesis according to at least one embodiment of the present disclosure includes a first tube assembly 21, a second tube assembly 22 and a physical position-limiting device 100.

In some examples, the first tube assembly 21 is configured for an artificial prosthesis to be placed therein. At least part of the second tube assembly 22 is sleeved on an outer side of at least part of the first tube assembly 21. The axial direction of the second tube assembly 22 and the axial direction of the first tube assembly 21 are parallel to or coaxial with the axial direction of the physical position-limiting device 100, respectively.

The physical position-limiting device 100 includes a first movable component 11, a guiding rod 12, a safety device 13 and a second movable component 14. Regarding the specific structure, construction and technical effects of the following physical position-limiting device 100 of the conveying system 200, reference can be made to the description of Figs. 1 to 6, which will not be repeated here for the sake of clarity and conciseness.

In some examples, at least part of the second tube assembly 22 (e.g., the sheath tube 221 described below) is fixedly connected with the first movable component 11, so that the first movable component 11 drives the second tube assembly 22 to move axially relative to the first tube assembly 21.

As mentioned above, the safety rod 131 is immovable relative to the current operator (or can be immovable relative to the current environment where the whole conveying system is located), so that the safety rod 131 is also immovable relative to, for example, the first tube assembly 21; that is, the first tube assembly 21 can also be immovable relative to the current operator.

In some examples, the physical position-limiting device 100 of the conveying system 200 further includes a screw 15. The first movable component 11 (for example, the first knob 111) is internally provided with a thread matched with the screw 15 to obtain a threaded fit, so that the first movable component 11 can carry out a relative movement in the axial direction through screw drive when the first movable component 11 performs a circumferential rotation, thereby driving the guiding rod 12 to move in the axial direction of the physical position-limiting device 100.

For example, the guiding rod 12 passes through the screw 15 and can move relative to the screw 15. The first movable component 11 (for example, the first knob 111) is sleeved on the screw 15, and the axial directions of the first movable component 11 and the screw 15 are both parallel to or coaxial with the axial direction of the guiding rod 12. The first movable component 11 (for example, the first knob 111) is fixedly connected with an intermediate component (for example, the sheath tube holder 28 described below), and the intermediate component (for example, the sheath tube holder 28 described below) is fixedly connected with the guiding rod 12, so that when the first movable component 11 (for example, the first knob 111) rotates, it can carry out a relative movement in the axial direction through screw drive and drive the intermediate component (for example, the sheath tube holder 28 described below) to move, so as to drive the guiding rod 12 to move along the axial direction of the physical position-limiting device 100.

In some examples, the conveying system 200 further includes a first housing including a first half shell 23 and a second half shell 24, and a second housing including a third half shell 25 and a fourth half shell 26.

For example, as shown in Fig. 7, the distal end of the screw 15 is fixedly connected with the first half shell 23 and the second half shell 24, respectively. The first half shell 23 and the second half shell 24 are located at both sides of the central axis of the conveying system 200 (i.e., the up-and-down direction in Fig. 7) respectively, and the first half shell 23 (i.e., the upper shell) and the second half shell 24 (i.e., the lower shell) are fixedly connected to form the first housing. For example, as shown in Fig. 7, the proximal end of the screw 15 is fixedly connected with the third half shell 25 and the fourth half shell 26, respectively. The third half shell 25 and the fourth half shell 26 are located at both sides of the central axis of the conveying system 200 (i.e., the up-and-down direction in Fig. 7) respectively, and the third half shell 25 (i.e., the upper shell) and the fourth half shell 26 (i.e., the lower shell) are fixedly connected to form the second housing.

In some embodiments of the present disclosure, the first housing and the second housing are each formed by assembling two half shells with each other, which facilitates the installation of the whole conveying system.

It should be noted that the present disclosure is not limited to this. For example, in some other examples, the first half shell 23 and the second half shell 24 may be located at the left and right sides of the axis of the conveying system 200, respectively, and/or, the third half shell 25 and the fourth half shell 26 may be located at the left and right sides of the axis of the conveying system 200, respectively. Alternatively, in still some other examples, the first housing and the second housing each have an integral structure.

In some examples, the first tube assembly 21 is fixedly connected with the second housing, and both the first tube assembly 21 and the second housing remain immovable (for example, immovable relative to the operator).

Detailed structures of the first tube assembly 21 and the second tube assembly 22 will be described below with reference to Fig. 8.

As shown in Fig. 8, the first tube assembly 21 includes an artificial prosthesis connector 211 and an inner tube 213. For example, the inner tube 213 is a multi-layered tube 213 to increase the strength, but the present disclosure is not limited to this. For the convenience of description, a multi-layered tube 213 is adopted for all the inner tubes 213 involved in the following.

In some examples, the artificial prosthesis connector 211 is arranged on an outer surface of at least part of the multi-layered tube 213 and is fixedly connected with the multi-layered tube 213, for example, through glue. For example, the artificial prosthesis connector 211 is arranged on the outer surface of the distal end of the multi-layered tube 213 and is fixedly connected with the multi-layered tube 213.

According to at least one embodiment of the present disclosure, the outer surface of the artificial prosthesis connector 211 is provided with a clamping slot, which is matched with the artificial prosthesis for the artificial prosthesis to be embedded therein and is detachably connected with the artificial prosthesis. For example, in some examples, the clamping slot on the outer surface of the artificial prosthesis connector 211 is a T-shaped groove, and the proximal end of the artificial prosthesis is processed with a T-shaped rod with the same shape. Of course, this is only exemplary and is not a limitation to the embodiment of the present disclosure, provided that the shape of the clamping slot on the artificial prosthesis connector 211 is matched with the shape of the artificial prosthesis.

In some examples, the artificial prosthesis includes, but is not limited to, an artificial heart valve. The present disclosure is neither limitative nor exhaustive. For example, in some other examples, the artificial prosthesis is a stent graft or an artificial blood vessel prosthesis for treating vascular diseases such as aneurysms.

In some examples, the first tube assembly 21 further includes an end base 212, and the end base 212 is connected with the distal end of the multi-layered tube 213. For example, the end base 212 is a conical head, which is located at the farthest end of the conveying system and detachably connected with the distal end of the multi-layered tube 213. For example, as shown in Fig. 9, the end base 212 and the distal end of the multi-layered tube 213 are fixedly connected by threads. This is merely exemplary and is not a limitation of the present disclosure.

In some examples, the multi-layered tube 213 includes a plurality of third tube bodies which are connected in sequence along the axial direction of the first tube assembly 21 and have different hardness. For example, the multi-layered tube 213 is composed of a plurality of layers and sections of polymer materials with different hardness, and hence has a plurality of axially connected straight sections with different hardness, so that the straight sections required to have certain strengths can possess sufficient strengths, and the straight sections required to be bent can possess sufficient flexibilities. In this way, the conveying system including such multi-layered tube 213 can be used to access the arterial arch (for example, with a U-like shape) to convey an artificial heart valve.

In some examples, the second tube assembly 22 includes a sheath tube 221 and a stabilizer tube 222, and the distal end of the stabilizer tube 222 is sleeved on an outer side of at least part of the sheath tube 221.

For example, as shown in Fig. 8, the sheath tube 221 is a reducer tube structure, which includes a first sheath tube portion 221a and a second sheath tube portion 221b arranged in sequence from the distal end to the proximal end, that is, the first sheath tube portion 221a is closer to the distal end than the second sheath tube portion 221b is to the distal end. The diameter of the first sheath tube portion 221a is larger than that of the second sheath tube portion 221b.

For example, the stabilizer tube 222 is sleeved on an outer side of at least part of the second sheath tube portion 221b, that is, the diameter of the stabilizer tube 222 is larger than that of the second sheath tube portion 221b. For the relationship between the diameter of the first sheath tube portion 221a and the diameter of the stabilizer tube 222, for example, the diameter of the stabilizer tube 222 is smaller than that of the first sheath tube portion 221a, which can ensure that the conveying system can be completely opened stably and accurately when the sheath tube 221 moves to the position where the first sheath tube portion 221a is caught by the distal end of the stabilizer tube 222.

In some examples, along the radial direction, the stabilizer tube 222, the second sheath tube portion 221b and the multi-layered tube 213 are sequentially arranged from the outside to the inside, as shown in Fig. 8.

In some examples, the sheath tube 221 includes a plurality of fourth tube bodies which are sequentially connected along the axial direction and have different hardness. For example, different tubing preparation methods are adopted for different straight sections of the sheath tube 221 to obtain a plurality of straight sections with different hardness, so that the straight sections required to have certain strengths can possess sufficient strengths and the straight sections required to be bent can possess sufficient flexibilities. In this way, a conveying system including such a sheath tube 221 can be used to access the arterial arch (for example, with a U-like shape) to convey an artificial heart valve.

For example, when loaded, the T-shaped rod of the artificial prosthesis is embedded in the T-shaped groove on the artificial prosthesis connector 211; subsequently, when the sheath tube 221 of the second tube assembly 22 is closed (that is, when the sheath tube 221 moves to the left side), the artificial prosthesis is loaded into the cavity formed by the sheath tube 221 and the first tube assembly 21. This is merely exemplary and is not a limitation of the present disclosure.

The above-described embodiment of the present disclosure provides the loading of the artificial prosthesis through the artificial prosthesis connector provided with the clamping slot, which is suitable for various artificial prostheses, ensures the smooth release of the artificial prosthesis, and has the advantages of simple structure, convenient operation and wider application.

The detailed structure of the conveying system 200 will be described below with reference to Fig. 9. As shown in Fig. 9, the first movable component 11 may be a manual first knob 111, for example. The axial direction of the first knob 111 is parallel to or coaxial with the axial direction of the guiding rod 12. For example, the guiding rod 12 is coaxial with the axis of the whole conveying system 200.

As shown in Fig. 9, the conveying system 200 further includes a stabilizer tube holder 27 basically arranged inside the first housing. The stabilizer tube holder 27 is arranged inside the first housing, and the stabilizer tube 222 extends from the distal end to the stabilizer tube holder 27, and the proximal end of the stabilizer tube 222 is fixedly connected with the stabilizer tube holder 27. The stabilizer tube holder 27 is fixedly connected with the first half shell 23 and the second half shell 24 which constitute the first housing, respectively. Thus, the stabilizer tube 222 and the stabilizer tube holder 27 are fixed relative to the first housing.

In some examples, the stabilizer tube holder 27 is fixedly connected with the stabilizer tube 222 through glue, and the stabilizer tube holder 27 is fixedly connected with the first half shell 23 and the second half shell 24 by means of a form fit, respectively. For example, the stabilizer tube holder 27 is cylindrical, and two ribs are processed inside the first half shell 23 (and/or the second half shell 24), and there are semicircular openings on the ribs for clamping the stabilizer tube holder 27 to achieve the form fit. This is merely exemplary and is not a limitation of the present disclosure.

In some examples, the conveying system 200 also includes a sheath tube holder 28. The sheath tube 221 extends from the distal end to the sheath tube holder 28, and the proximal end of the second sheath tube portion 221b of the sheath tube 221 is fixedly connected with the sheath tube holder 28. In some examples, the sheath tube holder 28 is fixedly connected with the first knob 111.

For example, the sheath tube holder 28 and the sheath tube 221 are fixedly connected through glue. For example, the sheath tube holder 28 is fixedly connected with the first knob 111 by means of form fit. For example, the guiding rod 12 and the sheath tube holder 28 are fixedly connected by threads and glue. This is merely exemplary and is not a limitation of the present disclosure.

As shown in Fig. 8, the conveying system 200 further includes an inner tube holder 29. For example, in the case where the inner tube 213 is a multi-layered tube 213, correspondingly, the inner tube holder 29 can also be referred to as a multi-layered tube holder 29. All the inner tube holders 29 referred to below are described as a multi-layered tube holder 29.

For example, the multi-layered tube 213 extends from the distal end to the proximal end until it reaches the multi-layered tube holder 29, which is fixedly connected with the multi-layered tube 213. The multi-layered tube holder 29 is arranged inside the second housing and is fixedly connected with the third half shell 25 and the fourth half shell 26 respectively, so that the first tube assembly 21 is fixedly connected with the third half shell 25 and the fourth half shell 26 respectively through a connector (for example, the multi-layered tube holder 29).

In some examples, the multi-layered tube holder 29 is fixedly connected with the multi-layered tube 213 through glue, and the multi-layered tube holder 29 is fixedly connected with the third half shell 25 and the fourth half shell 26 by means of form fit, respectively. For example, the third half shell 25 and the fourth half shell 26 are immovable with respect to the multi-layered tube holder 29 and the multi-layered tube 213. In some examples, the second housing formed by fixedly connecting the third half shell 25 and the fourth half shell 26 is a cylinder, and the second knob 141 is sleeved on the outer side of the cylinder, so that the second knob 141 cannot move relative to the second housing in the axial direction but can rotate around the axial direction.

According to the above embodiment of the present disclosure, the multi-layered tube holder 29 can be utilized to guarantee the immovable state of the first tube assembly, so that the second tube assembly 22 can move axially relative to the first tube assembly 21 to ensure the smooth conveying and recovery of the artificial prosthesis.

In some examples, the first tube assembly 21 further includes an inner tube evacuator 214. The inner tube evacuator 214 is fixedly connected with the proximal end of the multi-layered tube 213. The operator needs to evacuate the air in the first tube assembly 21 before operation and inject physiological saline into the conveying system 200 by using a syringe, i.e., the inner tube evacuator 214 provides the operator with an interface that can be matched with the syringe, which is convenient for the operator to evacuate. In some examples, the inner tube evacuator 214 and the multi-layered tube 213 are fixedly connected through glue. Of course, this is only an example, and the embodiment of the present disclosure does not limit the fixed connection mode between the components.

In some examples, the guiding rod 12 is arranged inside the screw 15 and fixedly connected with the sheath tube holder 28, so that the sheath tube holder 28 can be driven to move by the rotation of the first knob 111, thereby driving the sheath tube 221 and the guiding rod 12 to move in the axial direction.

Therefore, in a case that the sheath tube 221 of the second tube assembly 22 moves proximally in the axial direction along with the rotation of the first knob 111, since the stabilizer tube 222 is immovable (for example, relative to the operator) and the diameter of the stabilizer tube 222 is smaller than the diameter of a part of the first sheath tube portion 221a, when the sheath tube 221 moves to a position where the first sheath tube portion 221a is caught by the distal end of the stabilizer tube 222, it can be considered that the sheath tube 221 moves to the rightmost end; at this time, the guiding rod is also located at the rightmost end (e.g., the position where the guiding rod 12 is located when the safety rod 131 is located at the leftmost end of the third groove section 131c as shown in Fig. 5d). This is the state where the conveying system is completely opened.

When rotated, the first knob 111 can move along the axial direction of the conveying system 200 due to the characteristics of screw drive. Moreover, since the first knob 111 is fixedly connected with all of the sheath tube holder 28, the sheath tube 221 of the second tube assembly 22 and the guiding rod 12, when the first knob 111 moves along the axial direction, it will drive the sheath tube holder 28 to move along the axial direction, which will also drive the sheath tube 221 to move along the axial direction. Since the first tube assembly 21 is fixedly connected with the third half shell 25 and the fourth half shell 26 respectively, i.e., they all remain immovable relative to the operator, the sheath tube 221 of the second tube assembly 22 will move relative to the first tube assembly 21 in the axial direction, so as to complete the conveying and release of artificial prosthesis (such as a heart valve).

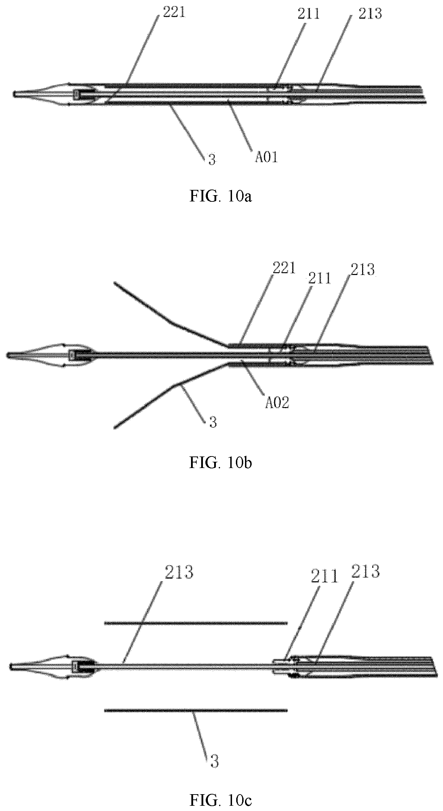

Figs. 10a-10c are schematic diagrams of an operation method of the conveying system for an artificial prosthesis provided by some embodiments of the present disclosure.

First of all, as shown in Fig. 10a, when the first end of the safety rod 131 is inserted into the first groove section 121a, at least part of (e.g., the whole prosthesis or a part of the prosthesis) an artificial prosthesis (e.g., a heart valve 3) is placed in a first cavity A01 between the sheath tube 221 of the second tube assembly 22 and the first tube assembly 21, and the first cavity A01 is gradually opened or closed while the sheath tube 221 of the second tube assembly 22 moving axially relative to the first tube assembly 21, so as to release the artificial prosthesis or recover the artificial prosthesis.

Secondly, as shown in Fig. 10b, when the first end of the safety rod 131 reaches the end inside the first groove section 121a close to the second groove section 121b (that is, the distal end inside the first groove section 121a), the artificial prosthesis is placed in a second cavity A02 between the sheath tube 221 of the second tube assembly 22 and the first tube assembly 21. As shown in Fig. 10b, when the first end of the safety rod 131 is limited in position at the distal end inside the first groove section 121a, a part of the artificial prosthesis (for example, 25% of the artificial prosthesis) is placed in the second cavity A02 between the sheath tube 221 of the second tube assembly 22 and the first tube assembly 21. At this time, the remaining part of the artificial prosthesis (for example, 75% of the artificial prosthesis) is released to the outside of the conveying system. The space of the second cavity A02 is smaller than that of the first cavity A01. Of course, the embodiment of the present disclosure does not limit the proportion of the released part of the artificial prosthesis when it reaches the limiting position, as long as it's satisfied that a part of the artificial prosthesis (that is, 0%~100% of the artificial prosthesis, excluding 0% and 100%) is located in the cavity. The relevant proportion of the released part of the artificial prosthesis may depend on different conditions of the artificial prosthesis, and will not be exhaustive or detailed here.

Thus, in the examples of Figs. 10a and 10b, the cavity formed between the sheath tube 221 of the second tube assembly 22 and the first tube assembly 21 is being opened, and the artificial prosthesis is being gradually released.

For example, when the conveying system 200 reaches the state shown in Fig. 10b, that is, when the guiding rod 12 is limited in position by the safety rod 131 of the safety device 13 and can no longer move along the axial direction, the operator can make a pause and judge whether the artificial prosthesis satisfies the target requirements instead of causing the artificial prosthesis to be completely released directly due to mis-operation, and the hand of the operator can temporarily leave the conveying system 200 to perform other surgical operations such as angiography.

The conveying system for conveying an artificial prosthesis in the above embodiment of the present disclosure has a physical position-limiting device, which can effectively feed the accurate information in release situation of the artificial prosthesis (e.g., whether the release length and release form satisfy the target requirements) back to the operator, and the operator can be reminded of position-limiting critical point through tactile sensation (e.g., reminding the operator of a critical point of position for recovering through tactile sensation), thereby realizing a simple, highly efficient and accurate physical limiting of position.

In some examples, when the artificial prosthesis is partially released, that is, when the guiding rod 12 is limited in position by the safety rod 131 of the safety device 13, if the operator judges that the artificial prosthesis satisfies the target requirements, the safety rod 131 is rotated from the distal end of the first groove section 121a to the third groove section 121c through the second groove section 121b by means of the axial rotation of the second movable component 14. At this time, the guiding rod 12 can be driven, by the first knob 111, to continue to move proximally along the axial direction, and then the safety rod 13 continues to slide, relative to the guiding rod 12, inside the third groove section 121c towards the distal end of the third groove section 121c along the axial direction until it reaches the farthest end inside the third groove section 121c. Moreover, when driven by the first movable component 11, the sheath tube 221 of the second tube assembly 22 moves axially relative to the first tube assembly 21, so that the second cavity A02 between the sheath tube 221 of the second tube assembly 22 and the first tube assembly 21 is further opened (that is, the space of the second cavity A02 becomes smaller and smaller), and more of the artificial prosthesis is released until the whole artificial prosthesis is completely released. That is, at this time, the artificial prosthesis is detached from the sheath tube 221, thereby completing the release and implantation of the artificial prosthesis, as shown in Fig. 10c.

In some examples, the artificial prosthesis is completely released when the safety rod 13 slides, relative to the guiding rod 12, to reach the farthest end of the third groove section 121c. This also means that the conveying system is completely opened at this time.

It should be noted that if the artificial prosthesis has been completely released before the safety rod 13 sliding relative to the guiding rod 12 reaches the farthest end of the third groove section 121c (for example, it's closer to the farthest end or at the middle position inside the third groove section 121c), it is also within the scope of the embodiments of the present disclosure, which is not limited in the embodiment of the present disclosure.

In some examples, after the safety rod 131 reaches the distal end inside the third groove section 121c and the artificial prosthesis is completely released, the first knob 111 is controlled to rotate reversely (that is, the first knob 111 also moves distally along the axial direction), which drives the sheath tube 221 of the second tube assembly 22 to move axially relative to the first tube assembly 21, and drives the guiding rod 12 to move distally along the axial direction, so that the safety rod 131 performs a relative movement, then returns into the first groove section 121a from the third groove section 121c through the fourth groove section 121d, and finally reaches the proximal end of the first groove section 121a, thereby realizing a complete closure of the conveying system .

In some examples, when the artificial prosthesis is partially released, that is, when the guiding rod 12 is limited in position by the safety rod 131 of the safety device 13, if the operator judges that the artificial prosthesis fails to satisfy the target requirements, the first knob 111 is rotated reversely to drive the sheath tube 221 of the second tube assembly 22 to move to the distal end so as to close the cavity, thereby realizing the recovery of the artificial prosthesis. At this time, the operator can move or adjust the position and orientation of the whole conveying system for artificial prosthesis or perform other surgical operations such as angiography, and repeat the release and recovery operations of the artificial prosthesis again until the target requirements are satisfied.

Thus, the conveying system in at least one embodiment of the present disclosure can achieve the release and implantation of the artificial prosthesis, and the recovery of the artificial prosthesis in the state of incomplete release, which allows to provide a more fault-tolerant space for the operator, thereby reducing the operation difficulty of the operator. The conveying system in at least one embodiment of the present disclosure can recover the artificial prosthesis for positioning and releasing again in case of bad release position or bad release form, and can continue to perform the release in case of good release position or good release form of the artificial prosthesis, thereby further increasing the release accuracy, improving the implantation effect of the artificial prosthesis, and also enhancing the safety of recovery.

Therefore, the conveying method for conveying an artificial prosthesis in at least one embodiment of the present disclosure can implement the functions of prosthesis conveying, prosthesis recovery, prosthesis release, as well as closure of the conveying system.

It should be noted that in the embodiment of the present disclosure, the conveying method (that is, the operation method of the conveying system for artificial prosthesis) may include more or less steps, and the sequence among various steps is not limited but may be determined according to actual needs. The conveying method is realized based on the conveying system of any of the embodiments above, and for the contents of solutions of the conveying system related to the conveying method, reference can be made to the description of related embodiments above, which will not be repeated here.

The following conveying method for conveying artificial prosthesis is mainly described with reference to the case where an artificial heart valve prosthesis is conveyed and the guiding rod of the conveying system is provided with one level of limiting slot, by way of example. However, the conveying method according to the present disclosure is not limited to this. The present disclosure is not intended to make any limitation thereto, so it is not exhaustive or detailed here.

In some examples, when the operator rotates the first knob 111 to drive the sheath tube 221 of the second tube assembly 22 to move towards the proximal end along the axial direction, the functions of conveying and releasing the heart valve can be implemented. When the operator reversely rotates the first knob 111 to drive the sheath tube 221 of the second tube assembly 22 to move towards the distal end along the axial direction, the functions of recovering the heart valve and closing the conveying system after release can be achieved.

In some examples, the operation method of conveying the artificial heart valve prosthesis includes one or more of the following processes.

For example, when the operator rotates the first knob 111, the first knob 111 can move along the axial direction of the guiding rod 12 due to the characteristics of screw drive, which drives the sheath tube holder 28 to move axially, and hence also drives the sheath tube 221 to move axially; then the sheath tube 221 of the second tube assembly 22 will move axially relative to first tube assembly 21.

For example, when the sheath tube 221 of the second tube assembly 22 moves towards the proximal end, the cavity formed by the sheath tube 221 and the first tube assembly 21 will be gradually opened, and the heart valve 3 inside the cavity will be gradually released. At the same time, the first knob 111 also drives the guiding rod 12 to move towards the proximal end, and the safety rod 131 inside the first groove section 121a of the guiding rod 12 slides relative to the guiding rod 12. With the movement of the guiding rod 12, the safety rod 131 reaches the distal end (as shown in Fig. 5b) inside the first groove section 121a from the proximal end (the heart valve 3 in an initial state shown in Fig. 5a is completely located in the cavity formed by the sheath tube 221 and the first tube assembly 21). At this time, due to the blocking effect of the inner wall of the first groove section 121a, when the first knob 111 is continuously operated, the guiding rod 12 is limited in position by the safety rod 131 inside the first groove section 121a and can no longer move further towards the proximal end, and the sheath tube 221 fixedly connected with the guiding rod 12 cannot move further towards the proximal end; as a result, the heart valve 3 cannot be further released. At this time, the operator can be reminded that this position is the limiting position for recovery of the heart valve 3 through tactile sensation. For example, at this time, 75% of the heart valve 3 is released, and 25% of the heart valve 3 is still in the cavity formed by the sheath tube 221 and the first tube assembly 21.

For example, when the operator judges that the heart valve 3 satisfies the target requirements (for example, the release form and release position of the heart valve 3 are good or normal), he/she can rotate the second knob 141 to drive the safety rod 131 to rotate from the distal end of the first groove section 121a into the third groove section 121c through the second groove section 121b. The operator can continue to rotate the first knob 111, then the guiding rod 12 can continue to move towards the distal end under the driving of the moving first knob 111, so that the safety rod 13 slides relative to the guiding rod 12 inside the third groove section 121c until it reaches the distal end of the third groove section 121c. Moreover, the cavity between the sheath tube 221 of the second tube assembly 22 and the first tube assembly 21 is further opened under the driving of the first knob 111, and the heart valve 3 is further released until the whole heart valve 3 is completely released, thus completing the release and implantation of the heart valve 3, as shown in Fig. 10c.

For example, when it is judged that the heart valve 3 fails to satisfy the target requirements (for example, the release form and release position of the heart valve 3 are not good or unnormal), the first knob 111 is reversely rotated (i.e., the first knob 111 also moves towards the distal end along the axial direction), and the sheath tube 221 of the second tube assembly 22 is driven to move towards the distal end to close the cavity, thereby realizing the recovery of the heart valve 3.

For example, when the sheath tube 221 moves to the closest end (i.e., the rightmost end), the guiding rod 12 also moves to the closest end, i.e., the safety rod 131 reaches the farthest end inside the third groove section 121c, and the conveying system is completely opened, as shown in Fig. 10c. In this state, if the conveying system needs to be closed, the operator can drive the sheath tube 221 and the guiding rod 12 to move towards the distal end along the axial direction by rotating the first knob 111. When the sheath tube 221 and the guiding rod 12 move to the middle position, the safety rod 131 transits from the third groove section 121c to the chute-shaped fourth groove section 121d, then reaches the first groove section 121a, and finally reaches the closest end of the first groove section 121a. In this way, the second knob 141 can be driven to passively rotate to the initial position (i.e., reset), at which time the sheath tube 221 moves to the farthest end (i.e. the leftmost end), and the conveying system is completely closed, i.e., it can be withdrawn to the outside of the patient's body.

According to the conveying method of at least one embodiment of the present disclosure, a physical position-limiting structure is arranged at a critical position in the release process, so that the operator can accurately stop the action when the artificial prosthesis is released to the critical position by utilizing tactile sensation and forcedly terminating the operation, without the need of observing the release length of the valve at all times. The conveying method of at least one embodiment of the present disclosure can also enable the operator to reset without additional operation of the second movable component when the conveying system is closed. In addition, the reset along the chute during the recovering process of the conveying system facilitates the operator to close the conveying system conveniently after completely releasing the valve prosthesis without additional operation, which makes the whole surgical procedure safer and more efficient.

It should be noted that in the embodiments of the present disclosure, for other related processes and technical effects of the conveying method, reference can be made to the above description of the conveying system, which will not be described here.

The following points need to be explained:
(1) The drawings of the embodiments of the present disclosure only relate to the structures involved in the embodiments of the present disclosure, and other structures can refer to the general design.
(2) In case of no conflict, the embodiments of the present disclosure and the features in the embodiments can be combined with each other to obtain new embodiment(s).

The above are only the specific implementations of the present disclosure, but the scope of protection of the present disclosure is not limited to this, and the scope of protection of the present disclosure shall be subject to the scope of protection of the claims.

## Claims

1. A physical position-limiting device, comprising:
a first movable component;
a guiding rod configured to be driven by the first movable component to move along an axial direction of the guiding rod, wherein an outer surface of the guiding rod is provided with at least one level of limiting slot, the at least one level of limiting slot extends generally along the axial direction of the guiding rod, each level of limiting slot comprises a first groove section, a second groove section and a third groove section which are sequentially communicated, and the second groove section is arranged at an angle relative to the first groove section and the third groove section respectively;
a safety rod comprising a first end and a second end which are opposite to each other along a lengthwise direction of the safety rod, wherein the first end is inserted into the limiting slot, and the safety rod is configured to be movable along the limiting slot relative to the guiding rod; and
a second movable component connected with the second end of the safety rod, wherein the second movable component is configured to drive, through rotation, the safety rod to rotate around the axial direction of the guiding rod, so that the first end of the safety rod moves along the second groove section.

2. The physical position-limiting device according to claim 1, wherein
the first groove section and the third groove section are configured to extend along the axial direction of the guiding rod, and the second groove section is configured to extend along a circumferential direction of the guiding rod, and the second groove section is located between the first groove section and the third groove section along the axial direction of the guiding rod.

3. The physical position-limiting device according to claim 1 or 2, wherein
the limiting slot further comprises a fourth groove section, which is configured for the first end of the safety rod to pass through, wherein a distal end of the fourth groove section is communicated with a proximal end of the third groove section, a proximal end of the fourth groove section is communicated with at least part of the first groove section, and the fourth groove section is not parallel with the second groove section.

4. The physical position-limiting device according to claim 3, wherein
the first groove section, the second groove section, the third groove section and the fourth groove section surround and delimit a trapezoid or a triangle.

5. The physical position-limiting device according to claim 3 or 4, wherein
the at least one level of limiting slot comprises N levels of limiting slots, where N is an integer greater than or equal to 2, and the first groove section of an i^{th} level of limiting slot is communicated with the third groove section of an (i-1)^{th} level of limiting slot, wherein a value i of each level is sequentially marked as 1, 2 ..., N from a proximal end to a distal end of the limiting slot.

6. A conveying system for an artificial prosthesis, comprising the physical position-limiting device according to any one of claims 1-5, and further comprising:
a first tube assembly configured for an artificial prosthesis to be placed therein; and
a second tube assembly comprising a sheath tube, wherein the sheath tube is sleeved on an outer side of at least part of the first tube assembly, and axial directions of the sheath tube and the first tube assembly are parallel to or coaxial with the axial direction of the guiding rod, respectively;
wherein the sheath tube is fixedly connected with the first movable component, so that the first movable component drives the sheath tube to move axially relative to the first tube assembly.

7. The conveying system according to claim 6, wherein
the safety rod is immovable relative to the first tube assembly.

8. The conveying system according to claim 6 or 7, further comprising a screw, wherein
the first movable component comprises a first knob which is sleeved on the screw, and axial directions of the first knob and the screw are both parallel to or coaxial with the axial direction of the guiding rod, and an interior of the first knob is provided with a screw thread matched with the screw to obtain a threaded fit so that the first knob carries out a relative movement along an axial direction of the first tube assembly through screw drive when the first knob rotates in a circumferential direction.

9. The conveying system according to claim 8, further comprising a first half shell, a second half shell, a third half shell and a fourth half shell, wherein
a distal end of the screw is fixedly connected with the first half shell and the second half shell respectively, and a proximal end of the screw is fixedly connected with the third half shell and the fourth half shell respectively, wherein the first half shell and the second half shell are respectively located at two sides across a central axis of the conveying system and are fixedly connected to form a first housing, and the third half shell and the fourth half shell are respectively located at two sides across the central axis of the conveying system and are fixedly connected to form a second housing.

10. The conveying system according to claim 9, wherein
the second tube assembly further comprises a stabilizer tube, and a distal end of the stabilizer tube is sleeved on an outer side of at least part of the sheath tube.

11. The conveying system according to claim 10, wherein
the sheath tube is a reducer tube comprising a first sheath tube portion and a second sheath tube portion which are sequentially arranged from a distal end to a proximal end of the sheath tube, wherein a diameter of the first sheath tube portion is larger than that of the second sheath tube portion, a distal end of the stabilizer tube is sleeved on an outer side of the second sheath tube portion, and a diameter of the stabilizer tube is smaller than that of at least part of the first sheath tube portion.

12. The conveying system according to claim 11, further comprising a stabilizer tube holder and a sheath tube holder, wherein
the stabilizer tube is configured to extend from a distal end of the stabilizer tube to the stabilizer tube holder, and a proximal end of the stabilizer tube is fixedly connected with the stabilizer tube holder; the stabilizer tube holder is arranged inside the first housing, and the stabilizer tube holder is fixedly connected with the first half shell and the second half shell respectively;
the sheath tube extends from a distal end of the sheath tube to the sheath tube holder, a proximal end of the second sheath tube portion is fixedly connected with the sheath tube holder, and the sheath tube holder is fixedly connected with the first knob;
the guiding rod passes through the screw and is fixedly connected with the sheath tube holder, so that the sheath tube holder can be driven to move by means of a rotation of the first knob, and the sheath tube and the guiding rod can be driven by means of the rotation of the first knob to move relative to each other along the axial direction of the first tube assembly.

13. The conveying system according to claim 11 or 12, wherein
the first tube assembly comprises an inner tube and an artificial prosthesis connector,
the artificial prosthesis connector is arranged on an outer surface of a distal end of the inner tube and is fixedly connected with the inner tube;
the artificial prosthesis connector is provided with a clamping slot, which is matched with the artificial prosthesis for the artificial prosthesis to be embedded and placed in the clamping slot and is detachably connected with the artificial prosthesis.

14. The conveying system according to claim 13, further comprising an inner tube holder, wherein
the inner tube is configured to extend proximally from a distal end of the inner tube to the inner tube holder and is fixedly connected with the inner tube holder, wherein the inner tube holder is arranged inside the second housing, and the inner tube holder is fixedly connected with the third half shell and the fourth half shell respectively.

15. The conveying system according to claim 13 or 14, wherein
the inner tube comprises a plurality of third tube bodies which are sequentially connected along the axial direction of the first tube assembly and have different hardness, and the sheath tube comprises a plurality of fourth tube bodies which are sequentially connected along the axial direction of the first tube assembly and have different hardness.

16. The conveying system according to any one of claims 13-15, wherein
the first tube assembly further comprises an inner tube evacuator and an end base, wherein the inner tube evacuator is connected with a proximal end of the inner tube, and the end base is connected with the distal end of the inner tube.

17. The conveying system according to any one of claims 9-16, further comprising a safety connector, wherein
the second movable component is a second knob, and an axial direction of the second knob is parallel to or coaxial with the axial direction of the guiding rod,
the safety connector is fixedly connected with the second end of the safety rod and the second knob respectively, so that the safety rod can be driven to rotate around the axial direction of the guiding rod by means of a rotation of the second knob.

18. The conveying system according to claim 17, wherein
the second housing is a cylinder, and the second knob is sleeved on an outer surface of the cylinder, so that the second knob cannot move along the axial direction of the guiding rod but can rotate around the axial direction of the guiding rod.

19. The conveying system according to any one of claims 6-18, wherein
the artificial prosthesis comprises an artificial heart valve, a stent graft or an artificial blood vessel.
